# EUROPÄISCHE PATENTANMELDUNG

(11) **EP 1 161 925 A1**
(43) Veröffentlichungstag der Anmeldung: **12.12.2001**
(21) Anmeldenummer: 01810442.2
(22) Anmeldetag: 04.05.2001
(51) Int. Cl.: A61B 17/02

(54) **Vorrichtung für den operativen Eingriff an einem Auge**

(30) Priorität: 31.05.2000 US 583517
(71) Anmelder: GRIESHABER & CO. AG SCHAFFHAUSEN, CH-8203 Schaffhausen (CH)
(72) Erfinder: Grieshaber, Hans R., 8200 Schaffhausen (CH); Maag, Werner, 8750 Glarus (CH); Althoff, Gerhard, 8185 Winkel (CH)
(74) Vertreter: Althoff, Gerhard

(57) **Zusammenfassung**

Die Erfindung bezieht sich auf eine Vorrichtung zur Verwendung beim Eingriff am Auge eines Lebewesens, mit einem in die Vorderkammer (V) des Auges (10) einführbaren und zum Zurückziehen der Regenbogenhaut (13) mit einem hakenförmig angeformten Einhängeteil (23) versehenen länglichen ersten Zugglied (20) oder mit einem in eine röhrchenförmige Sonde (40) eingezogenen sowie mit mindestens einem Einhängeteil (23) versehenen zweiten Zugglied (20').

Bei dem einzelnen Zugglied (20;20') ist mindestens das vordere als hakenförmiges Einhängeteil (23) ausgebildete Teilstück aus verformbarem Material mit Formgedächtniseffekt hergestellt, wobei das in die Vorderkammer (V) des Auges (10) eingeführte Einhängeteil (23) infolge der Materialeigenschaften zum Einhängen und Zurückziehen der Regenbogenhaut (13) wieder in die vorgegebene hakenförmige Form zurückführbar ist.

## Beschreibung

Die Erfindung bezieht sich auf eine Vorrichtung für den operativen Eingriff am Auge eines Lebewesens, mit einem in die Vorderkammer einführbaren und zum Zurückziehen der Regenbogenhaut mit mindestens einem hakenförmig ausgebildeten Einhängeteil versehenen Zugglied und einem in Längsrichtung daran verschiebbar angeordneten und mit der äusseren Kontur des Auges anliegend in Eingriff bringbaren Halteelement.

Für den operativen Eingriff an einem Auge ist zur Vergrösserung der Pupillenöffnung aus der EP-A 0 653 197 eine Vorrichtung in Form eines sogenannten Operationshakens bekannt, welche einen an mindestens einem Ende mit einem hakenförmig angeformten Einhängeteil versehenen und aus flexiblem Material hergestellten Führungskörper sowie ein daran in Längsrichtung verschiebbar angeordnetes und klemmend mit dem Führungskörper in Eingriff bringbares Halteelement umfasst. Zum Zurückziehen der Regenbogenhaut wird der Operationshaken durch einen Einschnitt (Inzision) in der Hornhaut (Cornea) in die Vorderkammer eingeführt und nach dem operativen Eingriff wieder herausgezogen.

Aus der US-A 5,716,328 ist eine weitere Vorrichtung in Form eines Operationshakens bekannt, welcher aus flexiblem Material hergestellt ist und zwei parallel zueinander angeordnete sowie am vorderen Ende jeweils mit einem hakenförmigen Einhängeteil versehene Führungskörper sowie ein daran angeordnetes und in axialer Richtung verschiebbares Halteelement umfasst. Bei diesem Operationshaken sind die beiden vorderen hakenförmigen Einhängeteile zum Erfassen und Zurückziehen eines möglichst grossen Bereichs der Regenbogenhaut im wesentlichen A-förmig gespreizt zueinander angeordnet.

Die Verwendung derartiger Operationshaken zur Vergrösserung der Pupillenöffnung für den operativen Eingriff am Auge eines Lebewesens hat sich bewährt. Bei den bekannten Operationshaken ist es jedoch erforderlich, dass der Einschnitt (Inzision) in der Hornhaut zur Vermeidung unbeabsichtigter Einrisse oder anderweitiger traumatischer Veränderungen mindestens so gross wie die gesamte Breite des angeformten hakenförmigen Einhängeteils ausgebildet ist.

Der Erfindung liegt somit die Aufgabe zugrunde für den operativen Eingriff am Auge eines Lebewesens eine Vorrichtung zur Vergrösserung der Pupillenöffnung zu schaffen, welche Vorrichtung einen wesentlich kleineren Einschnitt in der Hornhaut erfordert und problemlos in die Vorderkammer des Auges einführbar und wieder herausziehbar ist.

Gemäss der Erfindung wird diese Aufgabe dadurch gelöst, dass mindestens das vordere als Einhängeteil ausgebildete Teilstück des Zuggliedes aus verformbarem Material mit Formgedächtniseffekt hergestellt ist, und dass das Zugglied mit weitgehend geradlinig aufgebogenem Einhängeteil in die Vorderkammer einführbar und darin infolge der Materialeigenschaften zum Einhängen und Zurückziehen der Regenbogenhaut wieder in die vorgegebene hakenförmige Form zurückführbar ist.

Hierdurch ist es möglich, das Zugglied mit einem etwa geradlinig aufgebogenen Einhängeteil durch einen relativ kleinen Einschnitt in die Vorderkammer des Auges einzuführen, wobei das hakenförmige Einhängeteil nach dem operativen Eingriff beim Herausziehen infolge der eigenen Elastizität sukzessive aufgebogen wird und in weitgehend gestrecktem Zustand aus der Vorderkammer herausziehbar ist, ohne dass dabei der Einschnitt vergrössert oder aufgerissen wird.

Eine weitere Ausgestaltung der erfindungsgemässen Vorrichtung ist gekennzeichnet durch eine in die Vorderkammer einführbare längliche Sonde und ein mit weitgehend geradlinig aufgebogenem Einhängeteil derart in der Sonde angeordnetes Zugglied, dass bei einer in axialer Richtung orientierten Relativbewegung des Zuggliedes in Bezug auf die Sonde oder umgekehrt das Einhängeteil ausser Eingriff der Sonde gelangt und infolge der Materialeigenschaften zum Einhängen und Zurückziehen der Regenbogenhaut in die hakenförmige Form zurückführbar ist.

Hierdurch ist es möglich, die Sonde mit dem eingezogenen Zugglied durch einen relativ kleinen Einschnitt zum Einhängen und Zurückziehen der Regenbogenhaut in die Vorderkammer des Auges einzuführen und nach dem operativen Eingriff das Zugglied mit dem hakenförmigen Einhängeteil wieder in die Sonde zurückzuziehen. Bei dieser Variante wird die Sonde zusammen mit dem eingezogenen Zugglied aus der Vorderkammer herausgezogen, ohne dass dabei der Einschnitt in der Hornhaut vergrössert wird und sich folglich anschliessend selbstabdichtend verschliesst.

Weitere Merkmale der Erfindung ergeben sich aus der Beschreibung in Verbindung mit der Zeichnung und den Patentansprüchen.

Ausführungsbeispiele der Erfindung werden nachstehend anhand der Zeichnung beschrieben. Es zeigt:
- **Fig.1**: ein als Horizontalschnitt sowie in grösserem Massstab dargestelltes Teilstück eines Auges mit einem in gestrecktem Zustand in die Vorderkammer eingeführten Zugglied;
- **Fig.2**: das Teilstück des Auges gemäss Fig.1 mit einem am vorderen Ende des in die Vorderkammer eingeführten Zuggliedes selbsttätig angeformten Einhängeteil zum Zurückziehen der Regenbogenhaut;
- **Fig.3**: das Teilstück des Auges gemäss Fig.2 mit der zurückgezogenen Regenbogenhaut und das an der äusseren Kontur im Bereich des Limbus mit einem Halteelement fixierte Zugglied;
- **Fig.4**: das Teilstück des Auges gemäss Fig.3 mit dem in die Vorderkammer zurückgeschobenen Zugglied sowie in Bezug auf die Regenbogenhaut ausgehängt dargestellten Einhängeteil;
- **Fig.5**: das Teilstück des Auges gemäss Fig.4 mit dem weitgehend aus der Vorderkammer herausgezogenen Zugglied;
- **Fig.6**: das Teilstück des Auges gemäss Fig.1 mit einem zum Einführen und Herausziehen des Zuggliedes im Bereich des Limbus angeordneten Hülsenkörper;
- **Fig.7**: das Teilstück des Auges gemäss Fig.1 mit einer ersten Variante des zusammen mit einer röhrchenförmigen Sonde in die Vorderkammer eingeführten Zuggliedes;
- **Fig.8**: das Teilstück des Auges gemäss Fig.7 mit dem am vorderen Ende aus der Sonde ragenden Zugglied und dem angeformten Einhängeteil;
- **Fig.9**: das Teilstück des Auges gemäss Fig.8 mit der zurückgezogenen Regenbogenhaut sowie die an der äusseren Kontur im Bereich des Limbus mit einem Halteelement fixierte röhrchenförmige Sonde;
- **Fig.10**: das in Ansicht und in grösserem Massstab dargestellte erste Zugglied gemäss Fig.1 mit dem in Längsrichtung daran verschiebbar angeordneten Halteelement;
- **Fig.11**: das in Draufsicht dargestellte und mit dem Halteelement versehene erste Zugglied gemäss Fig.10;
- **Fig.12**: das gemäss der in Fig.11 eingezeichneten Linie XIIXII im Profilquerschnitt dargestellte und kreisförmig ausgebildete Zugglied gemäss Fig.10;
- **Fig.12A**: das als Variante dargestellte und im Profilquerschnitt elliptisch ausgebildete Zugglied gemäss Fig.10;
- **Fig.13**: den im Schnitt sowie in grösserem Massstab dargestellten und gemäss Fig.6 in die Hornhaut einsetzbaren Hülsenkörper;
- **Fig.14**: eine in Ansicht sowie in grösserem Massstab dargestellte Variante des koaxial in der röhrchenförmigen Sonde angeordneten Zuggliedes;
- **Fig.15**: das in Ansicht dargestellte und teilweise aus der röhrchenförmigen Sonde herausgeschoben dargestellte Zugglied gemäss Fig.14 mit dem angeformten Einhängeteil;
- **Fig.16**: eine in Draufsicht sowie in grösserem Massstab dargestellte Variante des in der röhrchenförmigen Sonde angeordneten Zuggliedes;
- **Fig.17**: das in Draufsicht dargestellte Teilstück des Auges mit der beispielsweise mittels zwei im Abstand zueinander angeordneter Zugglieder gemäss Fig.10 und Fig.15 zurückgezogenen Regenbogenhaut; und
- **Fig.18**: das in Draufsicht dargestellte Teilstück des Auges mit der anhand des Zugglieds gemäss Fig.16 zurückgezogen dargestellten Regenbogenhaut.

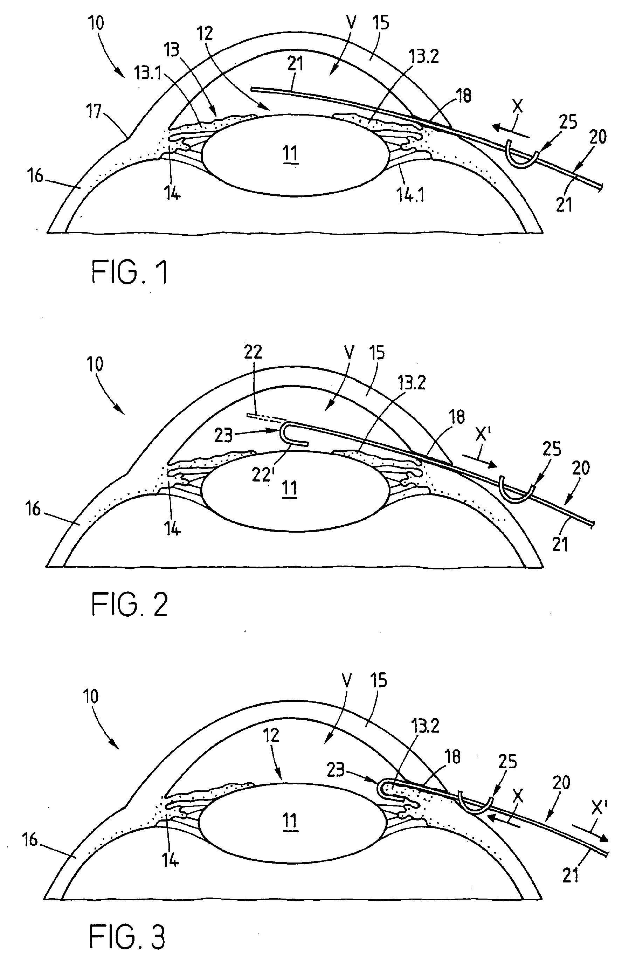

Zur Verdeutlichung der Erfindung ist in Fig.1 ein schematisch und als Horizontalschnitt dargestelltes Teilstück eines Auges 10 in grösserem Massstab dargestellt und man erkennt die Vorderkammer V, die Linse 11 (OKULAR), die Pupille 12, die Teilstücke 13.1 und 13.2 der Regenbogenhaut 13 (IRIS), die Zonulafasern 14.1 sowie den/die Ziliarkörper 14. Weiterhin ist in Fig.1 mit 15 die Hornhaut (CORNEA), mit 16 die Lederhaut (SKLERA) und mit 17 der Übergang (LIMBUS CORNEAE) von der Hornhaut 15 zur Lederhaut 16 bezeichnet. Zum Einführen eines in Fig.1 in der Gesamtheit mit 20 bezeichneten ersten Zuggliedes ist im Bereich des Übergangs 17 in der Hornhaut 15 ein Einschnitt 18 (Inzision) vorgesehen, welcher mit nicht dargestellten Mitteln in die Hornhaut 15 eingeschnitten wird. In den nachstehenden Figuren 2 bis 9 sind lediglich die jeweils relevanten Bezugszahlen des Auges 10 angegeben.

Das in Fig.1 schematisch dargestellte und in gestreckter Lage gemäss Pfeilrichtung X durch den Einschnitt 18 in die Vorderkammer V eingeführte Zugglied 20 umfasst einen länglichen flexiblen Führungskörper 21 sowie ein in Längsrichtung daran verschiebbar und klemmend angeordnetes Halteelement 25. Bei dem Führungskörper 21 ist mindestens das vordere in die Vorderkammer V einführbare Teilstück, vorzugsweise aber der gesamte längliche Führungskörper 21 aus elastisch verformbarem Material hergestellt. Infolge der besonderen Materialeigenschaften des länglichen Führungskörpers 21, insbesondere aber des in die Vorderkammer eingeführten vorderen Teilstücks 22 (Fig.2) ist dieses unter Berücksichtigung des eigenen Formgedächtniseffektes (SHAPE MEMORY) aus der in Fig.1 dargestellten gestreckten Form in eine ursprünglich vorgegebene Form, vorzugsweise in eine als hakenförmiges Einhängeteil 23 (Fig.2) ausgebildete Form zurückführbar.

Fig.2 zeigt das schematisch dargestellte Teilstück des Auges 10 gemäss Fig.1 mit dem in die Vorderkammer V eingeführten Zugglied 20, bei welchem in dieser Phase das vordere Teilstück 22 des Führungskörpers 21 selbsttätig aus der gestreckten Form in die ursprünglich vorgegebene und als Einhängeteil 23 etwa hakenförmig ausgebildete Form zurückgeführt ist. Sobald das vordere Teilstück 22 wieder die Form des Einhängeteils 23 aufweist, wird dieses in nicht dargestellter Weise mit dem zugeordneten Teilstück 13.2 der Regenbogenhaut 13 in Eingriff gebracht und gemäss Pfeilrichtung X' zurückgezogen. Das Zugglied 20 mit dem von dem Einhängeteil 23 erfassten Teilstück 13.2 kann in jeder beliebigen Position mittels dem an dem Führungskörper 21 klemmend angeordneten Halteelement 25 fixiert werden.

In Fig.3 ist das von dem Einhängeteil 23 des Zuggliedes 20 erfasste Teilstück 13.2 der Regenbogenhaut 13 beispielsweise in der maximalen äusseren Position dargestellt und mit dem in Pfeilrichtung X am Führungskörper 21 verschobenen und an der äusseren Kontur des Auges 10 im Bereich des Übergangs 17 (Limbus) anliegenden Halteelement 25 fixiert.

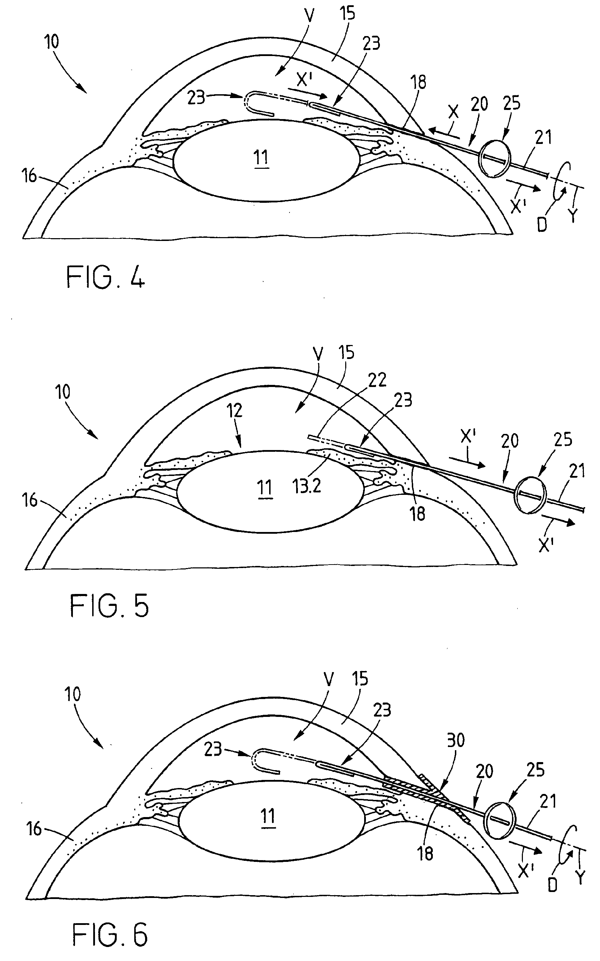

Nach dem operativen Eingriff (Fig.4) wird zunächst das Halteelement 25 gelöst und durch eine in Pfeilrichtung X' orientierte Bewegung am Führungskörper 21 verschoben. Anschliessend wird das Zugglied 20, wie in Fig.4 schematisch dargestellt, zunächst gemäss Pfeilrichtung X wieder in die Vorderkammer V zurückgeschoben. Hierbei wird das hakenförmige Einhängeteil 23 ausser Eingriff des Teilstücks 13.2 der Regenbogenhaut 13 gebracht, welches sich anschliessend und selbsttätig wieder in die natürliche Lage bewegt (Fig.4). Zum Herausziehen des Zuggliedes 20 aus der Vorderkammer V wird der längliche Führungskörper 21 vorzugsweise gemäss Pfeilrichtung D um einen Winkel von etwa 90° um die eigene Längsachse Y gedreht (Fig.4). Hierdurch wird erreicht, dass das hakenförmige Einhängeteil 23 beim Herausziehen gemäss Pfeilrichtung X' (Fig.5) ausser Eingriff des Teilstücks 13.2 der Regenbogenhaut 13 bleibt. Aufgrund der eigenen Elastizität wird das Einhängeteil 23 beim Herausziehen gemäss Pfeilrichtung X' sukzessive aufgebogen und mit etwa gestrecktem Teilstück 22, wie in Fig.5 schematisch dargestellt, aus der Vorderkammer V herausgezogen.

Bei einer in Fig.6 schematisch dargestellten Variante ist in dem Einschnitt 18 (Inzision) der Hornhaut 15 zusätzlich ein Hülsenkörper 30 angeordnet. Der Hülsenkörper 30 gewährleistet einerseits ein exaktes Einführen des gestreckten Führungskörpers 21 in die Vorderkammer V und dient andererseits als Anlage oder Gegenlager für das beim Herausziehen wieder in die gestreckte Lage aufzubiegende Einhängeteil 23. Mit dem eingesetzten Hülsenkörper 30 wird insbesondere beim Herausziehen des Zuggliedes 20 ein Verletzen der Kammer-Innenwand (nicht bezeichnet) durch das in dieser Phase noch nicht vollständig gestreckte Einhängeteil 23 verhindert.

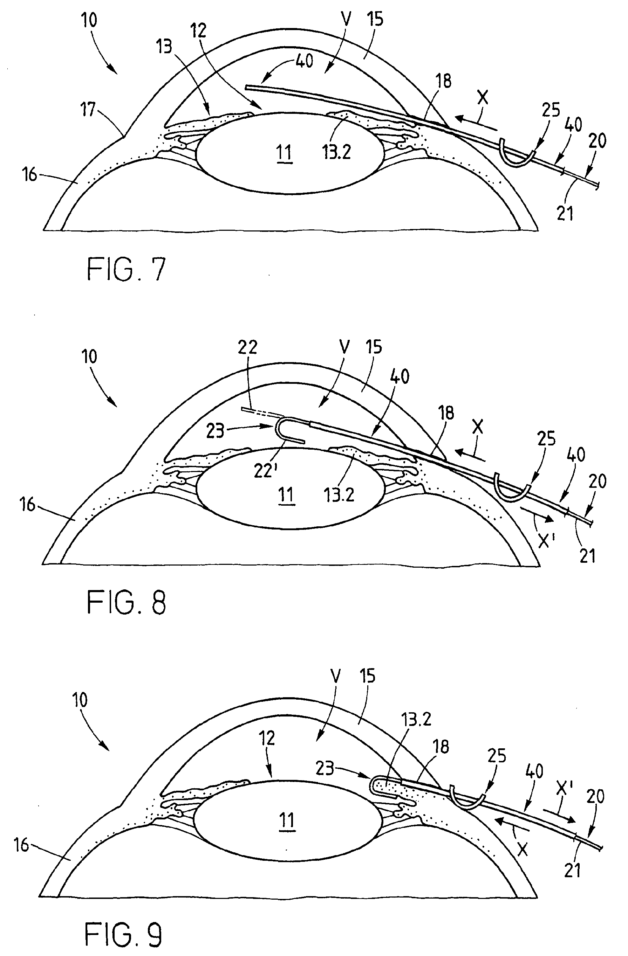

Bei einem weiteren Ausführungsbeispiel gemäss Fig.7 wird in einer ersten, nicht näher dargestellten Phase der längliche Führungskörper 21 des Zuggliedes 20 in eine röhrchenförmige Sonde 40 eingezogen und dabei das hakenförmig angeformte vordere Einhängeteil 23 entgegen der eigenen federelastischen Rückstellkraft zwangsläufig gerade gebogen. In dieser Stellung wird die Sonde 40, wie in Fig.7 dargestellt, mit dem koaxial darin angeordneten Führungskörper 21 gemäss Pfeilrichtung X durch den Einschnitt 18 (Inzision) in die Vorderkammer V des Auges 10 eingeführt. Bei diesem Ausführungsbeispiel wird die Sonde 40 mittels des am äusseren Umfang daran in axialer Richtung verschiebbare und an der äusseren Kontur des Auges 10 im Bereich des Übergangs 17 satt anliegenden Halteelements 25 fixiert.

Fig.8 zeigt das schematisch dargestellte Teilstück des Auges 10 gemäss Fig.7 mit der in die Vorderkammer V eingeführten röhrchenförmigen Sonde 40 sowie den koaxial darin angeordneten Führungskörper 21. In dieser Phase (Fig.8) wird durch die in Pfeilrichtung X' orientierte Relativbewegung der Sonde 40 in Bezug auf den Führungskörper 21 das vordere, schematisch dargestellte Teilstück 22 freigegeben und selbsttätig in die ursprüngliche als Einhängeteil 23 ausgebildete Form zurückgeführt.

Anschliessend wird das am vorderen Ende des Führungskörpers 21 angeformte Einhängeteil 23 in nicht dargestellter Weise mit dem zugeordneten Teilstück 13.2 der Regenbogenhaut 13 in Eingriff gebracht und infolge der in Pfeilrichtung X' orientierten Bewegung der beiden Teile 20 und 40 zurückgezogen. Auch bei diesem Ausführungsbeispiel wird das von dem Einhängeteil 23 erfasste Teilstück 13.2 in jeder beliebigen Position mittels des an der Sonde 40 in Pfeilrichtung X verschiebbaren und klemmend gehaltenen Halteelements 25 fixiert.

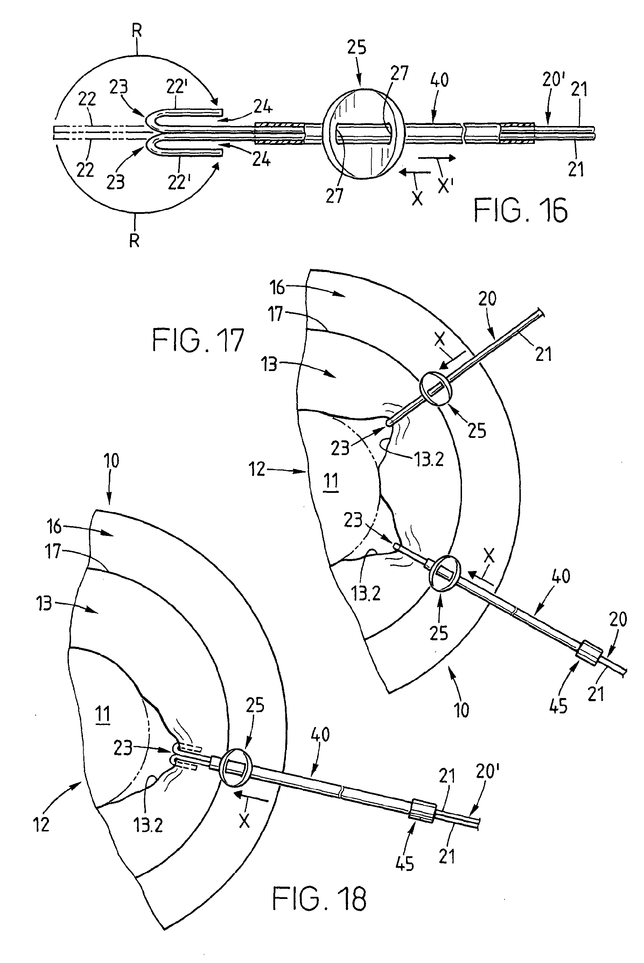

In Fig.9 ist die Sonde 40 mit dem koaxial darin angeordneten Führungskörper 21 mit dem Teilstück 13.2 der Regenbogenhaut 13 für den operativen Eingriff beispielsweise in der maximalen äusseren Position dargestellt und mit dem an der Sonde 40 verschiebbaren Halteelement 25 im Bereich des Übergangs 17 (Limbus) an der äusseren Kontur des Auges 10 fixiert. In dieser Position (Stellung), d.h. bei zurückgezogener Regenbogenhaut 13 wird der Führungskörper 21 beispielsweise durch die Reibung an der Innenwand der röhrchenförmigen Sonde 40 oder durch andere geeignete Mittel (Fig.17 und Fig. 18) derart gehalten, dass der Führungskörper 21 nicht selbsttätig infolge der Rückstellkraft des zusammengedrückten Teilstücks 13.2 der Regenbogenhaut 13 in die Vorderkammer V zurückgezogen wird.

Bei dem in den Figuren 7 bis 9 dargestellten Ausführungsbeispiel wird nach dem operativen Eingriff zunächst das Halteelement 25 gemäss Pfeilrichtung X' (Fig.8) wieder zurückgezogen und anschliessend die Sonde 40 zusammen mit dem Zugglied 20 gemäss Pfeilrichtung Z (Fig.8) in die Vorderkammer V geschoben. Sobald das hakenförmige Einhängeteil 23 ausser Eingriff des Teilstücks 13.2 der Regenbogenhaut 13 ist wird der Führungskörper 21 mit dem Einhängeteil 23 wieder in die röhrchenförmige Sonde 40 und diese anschliessend gemäss Pfeilrichtung X' aus der Vorderkammer V gezogen. Dieses Ausführungsbeispiel hat den Vorteil, dass die Sonde 40 zusammen mit dem eingezogenen Führungskörper 21 aus der Vorderkammer V entfernt werden kann. Ein Aufbiegen des hakenförmigen Einhängeteils 23 im Bereich des nicht näher bezeichneten Kammerwinkels (angulus irido-cornealis) der Vorderkammer V ist bei diesem Ausführungsbeispiel nicht erforderlich.

Die Verwendung des einzelnen Zuggliedes 20 gemäss Fig.1 bis Fig.6 aus dem verformbaren Material mit thermischem oder mechanischem Formgedächtniseffekt (SHAPE MEMORY) hat den besonderen Vorteil, dass der Einschnitt 18 (Inzision) in der Hornhaut 15 (Cornea) zum Einführen des Zuggliedes 20 lediglich etwa 0,2mm bis 0,4mm gross ist. Der Einschnitt 18 wird beispielsweise mit einem geeigneten OP-Instrument in Form eines Messers oder dergleichen durchgeführt. Der relativ kleine Einschnitt 18 hat den Vorteil, dass dieser sich nach dem Herausziehen des Zuggliedes 20 aus der Vorderkammer V ohne zusätzliche Hilfsmittel selbsttätig wieder verschliessen kann.

Bei dem Ausführungsbeispiel gemäss Fig.7 bis Fig.9 hat der in der röhrchenförmigen Sonde 40 angeordnete Führungskörper 21 des Zugglieds 20 einen Durchmesser von etwa 0,15mm und die vorzugsweise dünnwandig ausgebildete Sonde 40 einen äusseren Durchmesser von etwa 0,25mm bis 0,35mm. Auch hierbei ist der erforderliche Einschnitt 18 in der Hornhaut 15 ebenfalls relativ klein, so dass das selbsttätige Verschliessen desselben gewährleistet ist.

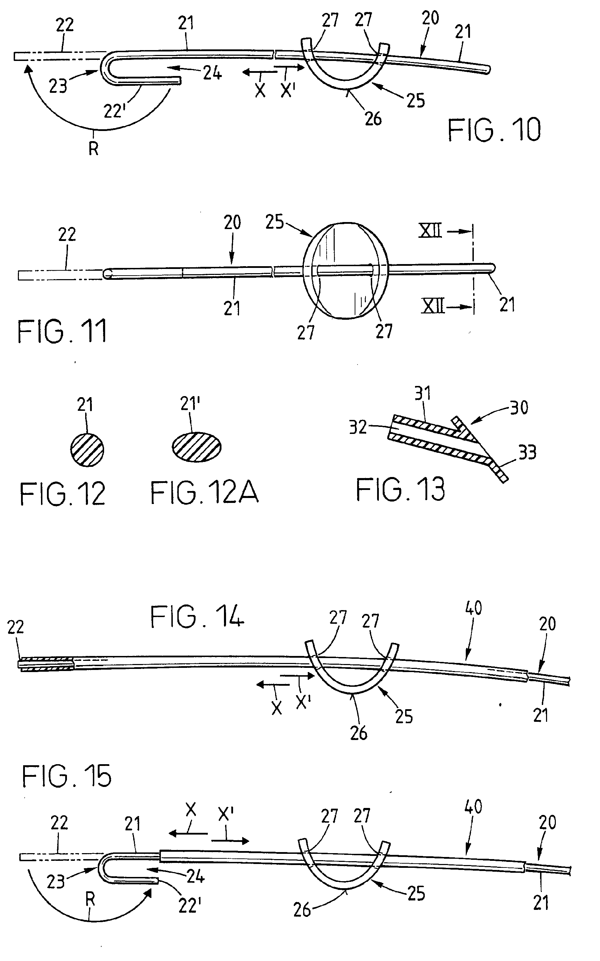

In Fig.10 ist das Zugglied 20 mit dem klemmend an dem Führungskörper 21 angeordneten Halteelement 25 in grösserem Massstab sowie in Ansicht und in Fig.11 in Draufsicht dargestellt. Das Zugglied 20 besteht aus dem länglichen flexiblen Führungskörper 21, welcher mindestens an dem einen Ende das einmal haarnadelförmig umgebogene Einhängeteil 23 aufweist. Das Einhängeteil 23 hat einen durch einen Spalt 24 im Abstand zu dem Führungskörper 21 angeordneten Schenkel 22'. Das an dem länglichen Führungskörper 21 in Pfeilrichtung X oder X' verschiebbar angeordnete Halteelement 25 ist beispielsweise als kreisförmige Scheibe ausgebildet, welche zum Aufschieben auf den Führungskörper 21 mit zwei im Abstand zueinander angeordneten Bohrungen 27 versehen ist. Das Halteelement 25 hat in aufgeschobenem und klemmend gehaltenen Zustand eine etwa bogenförmige Aussenseite 26, welche ein sattes Anliegen des Halteelements 25, wie beispielsweise in Fig.3 und Fig.9 dargestellt, im Bereich des Übergangs 17 an der äusseren Kontur des Auges 10 gewährleistet.

Zum Einführen des Zuggliedes 20 in die Vorderkammer V des Auges 10 (Fig.1) wird das an dem Führungskörper 21 angeformte Einhängeteil 23 mit dem Schenkel 22' beispielsweise bei einer auf die Temperatur in der Vorderkammer bezogenen tieferen Temperatur geradlinig aufgebogen und innerhalb der Vorderkammer infolge der höheren Temperatur und des Formgedächtniseffektes in die hakenförmige Form zurückgeführt. Beispielsweise wird das Einhängeteil 23 bei einer Raumtemperatur von etwa 20°C im wesentlichen gemäss Pfeilrichtung R (Fig.10) weitgehend gerade gebogen. Nach dem Einführen in die Vorderkammer V des Auges 10 wird das aufgebogene Einhängeteil 23 aufgrund der speziellen Materialeigenschaften (Formgedächtniseffekt) und durch die in der Vorderkammer V des Auges 10 vorhandene Temperatur (Körpertemperatur) von etwa 37°C sukzessive und selbsttätig wieder in die hakenförmige und als Einhängeteil 23 ausgebildete Form zurückgeführt (Fig.2). In dieser Stellung ist der Schenkel 22' etwa parallel im Abstand 24 zu dem Führungskörper 21 angeordnet und die ursprüngliche Form des Einhängeteils 23 wieder hergestellt.

Fig.12 zeigt den gemäss der Linie XII-XII in Fig.11 im Profilquerschnitt kreisförmig und Fig.12A den als Variante im Profilquerschnitt ellipsenförmig ausgebildeten Führungskörper 21, wobei die speziellen Materialien und farblichen Ausgestaltungen etc. nachstehend noch angegeben werden.

In Fig.13 ist der gemäss Fig.6 in den Einschnitt 18 der Hornhaut 15 einsetzbare Hülsenkörper 30 im Schnitt sowie in grösserem Massstab dargestellt. Der Hülsenkörper 30 umfasst ein Röhrchen 31 sowie einen an dem einen Ende desselben angeordneten Flansch 33. Der Flansch 33 ist zur Erreichung einer abdichtenden Anlage an der Hornhaut 15 (Fig.6) in Bezug auf die theoretische Längsachse einer den Hülsenkörper 30 in axialer Richtung durchdringenden Bohrung 32 geneigt an dem Röhrchen 31 angeordnet.

Fig.14 zeigt als weiteres Ausführungsbeispiel die in grösserem Massstab dargestellte und zur Aufnahme des Zuggliedes 20 ausgebildete Sonde 40 mit dem am äusseren Durchmesser desselben gemäss Pfeilrichtung X und X' verschiebbar angeordneten Halteelement 25. Das Zugglied 20 mit den einzelnen Elementen 21,22 und 23 sowie das mit den beiden im Abstand zueinander angeordneten Bohrungen 27 und der bogenförmigen Aussenseite 26 versehene Halteelement 25 sind analog dem vorstehend in Verbindung mit Fig.10 und 11 beschriebenen Elementen ausgebildet. Bei dem in Fig.14 dargestellten Ausführungsbeispiel ist der Führungskörper 21 mit dem angeformten Einhängeteil 23 und Schenkel 22' in die am vorderen Ende teilweise im Schnitt dargestellte röhrchenförmige Sonde 40 gezogen. In der eingezogenen Stellung des Zuggliedes 20 kann die Sonde 40, wie in Fig.7 dargestellt, problemlos durch den in der Hornhaut 15 vorgesehenen Einschnitt 18 in die Vorderkammer V des Auges 10 eingeführt werden.

Fig.15 zeigt die Sonde 40 gemäss Fig.14 bei welcher entweder das Zugglied 20 gemäss Pfeilrichtung X aus der Sonde 40 herausgeschoben oder die Sonde 40 gemäss Pfeilrichtung X' relativ zu dem Zugglied 20 zurückgezogen dargestellt ist. Der dabei etwa in gestreckter Lage (Strich-Punkt-Punkt-Strich-Linie) aus der röhrchenförmigen Sonde 40 herausgeschobene Führungskörper 21 wird dabei infolge des thermischen oder mechanischen Formgedächtniseffektes (SHAPE MEMORY) aus der schematisch gestreckt dargestellten Position sukzessive gemäss Pfeilrichtung R wieder in die ursprünglich vorgegebene und als hakenförmiges Einhängeteil 23 ausgebildete Form zurückgeführt. In dieser Stellung ist der Schenkel 22', wie in Fig.15 dargestellt, in etwa parallelem Abstand 24 zu dem Führungskörper 21 angeordnet. Für die in Pfeilrichtung X oder X' orientierte Relativbewegung des Zuggliedes 20 in Bezug auf die röhrchenförmige Sonde 40 kann zur besseren Handhabung an dem einen dem Einhängeteil 23 gegenüberliegenden Ende des Führungskörpers 21 ein Griffelement (nicht dargestellt) oder dergleichen angeordnet werden.

Bei dem in Fig.16 in grösserem Massstab dargestellten Ausführungsbeispiel ist in der röhrchenförmigen Sonde 40 ein mit zwei parallel zueinander angeordneten Führungskörpern 21 versehenes Zugglied 20' angeordnet. Auch bei diesem Ausführungsbeispiel wird entweder das Zugglied 20' mit den beiden Führungskörpern 21 gemäss Pfeilrichtung X aus der röhrchenförmigen Sonde 40 herausgeschoben oder die Sonde 40 gemäss Pfeilrichtung X' relativ zu dem Zugglied 20' zurückgezogen. Die beiden etwa in gestreckter Lage aus der Sonde 40 herausgeschobenen Führungskörper 21 werden infolge des thermischen oder mechanischen Formgedächtniseffektes (SHAPE MEMORY) aus der schematisch dargestellten gestreckten Form sukzessive gemäss Pfeilrichtung R wieder in die ursprünglich vorgegebene und als hakenförmiges Einhängeteil 23 ausgebildete Form zurückgeführt. In dieser Position sind die beiden Schenkel 22' zu dem jeweiligen Führungskörper 21, wie in Fig.16 dargestellt, in etwa parallelem Abstand 24 angeordnet.

Die beiden Einhängeteile 23 gemäss dem in Fig.16 dargestellten Ausführungsbeispiel sind mit den jeweils durch den Spalt 24 etwa parallel zum Führungskörper 21 angeordneten Schenkeln 22' unter spitzem Winkel etwa A-förmig gespreizt zueinander angeordnet. Zum Einführen der Sonde 40 in die Vorderkammer V des Auges 10 werden die beiden Führungskörper 21 mit den daran angeformten Einhängeteilen 23 in nicht dargestellter Weise gemäss Pfeilrichtung X' in die röhrchenförmige Sonde 40 gezogen.

Bei den vorstehend beschriebenen Führungskörpern 21 der einzelnen Zugglieder 20 oder 20' ist mindestens das vordere hakenförmige Einhängeteil 23 zum weitgehend geradlinigen Aufbiegen beziehungsweise zum selbsttätigen Zurückführen in die vorgegebene Form aus verformbaren Material mit thermischem oder mechanischem Formgedächtniseffekt (SHAPE MEMORY) hergestellt. Bei einem bevorzugten Ausführungsbeispiel ist jedoch der Führungskörper 21 über die gesamte Länge aus verformbarem Material mit thermischem oder mechanischem Formgedächtniseffekt in Form eines flexiblen Filaments oder Fadens hergestellt. Gemäss einer ersten Variante ist der einzelne Führungskörper 21 aus flexiblem, polymerem oder thermoplastischem Material mit thermischem oder mechanischem Forgedächtniseffekt hergestellt. Bei einer zweiten Variante kann der einzelne Führungskörper 21 auch aus flexiblem Metall, vorzugsweise aus einer Nickel-Titan Legierung mit sogenannter "Super-Elastizität" mit thermischem oder mechanischem Formgedächtniseffekt hergestellt werden. Die in Form eines flexiblen Filaments oder Fadens mit thermischem oder mechanischem Forgedächtniseffekt ausgebildeten Führungskörper 21 des jeweiligen Zuggliedes 20 oder 20' haben beispielsweise einen Durchmesser von etwa 0,12 mm bis 0,20 mm.

Bei einer weiteren Variante ist der einzelne Führungskörper 21 vorzugsweise ausgehend von dem Einhängeteil 23 (gestreckt oder hakenförmig verformt) in axialer Richtung gesehen bis mindestens zur Hälfte der gesamten Länge, vorzugsweise aber über die gesamte Länge aus einem in Bezug auf die Farbe der jeweiligen Regenbogenhaut 13 (Fig.17 und Fig.18) kontrastfarbenen Material, vorzugsweise aus einem optisch signalfarbenen Material hergestellt. Vorzugsweise hat der einzelne Führungskörper 21 einen in Bezug auf die Regenbogenhaut 13 homogen kontrastfarben eingefärbten kreisförmigen oder elliptischen Profilquerschnitt (Fig.12 und Fig.12A). Das Halteelement 25 ist ebenfalls aus einem elastisch verformbaren Material, beispielsweise aus einer signalfarben eingefärbten Silikon-Kautschukmasse hergestellt.

In Fig.17 ist ein Teilstück des Auges 10 in Draufsicht dargestellt und man erkennt das eine Ausführungsbeispiel des Zuggliedes 20 mit dem an der Regenbogenhaut 13 eingehängten Führungskörper 21 gemäss Fig.10 und Fig.11. Das Zugglied 20 ist dabei mit dem daran angeordneten und in Pfeilrichtung X verschiebbaren Halteelement 25 an der äusseren Kontur des Auges 10 beziehungsweise am Übergang 17 (Limbus) fixiert und gehalten.

Weiterhin erkennt man in Fig.17 das andere Ausführungsbeispiel gemäss Fig.14 und Fig.15, bei welchem der mit dem Einhängeteil 23 an der Regenbogenhaut 13 eingehängte Führungskörper 21 des Zuggliedes 20 koaxial in der Sonde 40 angeordnet ist. Die Sonde 40 ist hierbei mit dem ersten in Pfeilrichtung X daran verschiebbaren Halteelement 25 an der äusseren Kontur des Auges 10 beziehungsweise am Übergang 17 (Limbus) fixiert und gehalten. Das an der Regenbogenhaut 13 eingehängte Zugglied 20 ist mit einem zweiten in Pfeilrichtung X am Führungskörper 21 verschiebbaren Stopperelement 45 an der zugewandten Stirnseite (nicht bezeichnet) der Sonde 40 fixiert und gehalten.

Fig.18 zeigt ein weiteres Ausführungsbeispiel mit dem in Draufsicht dargestellten Teilstück des Auges 10 und dem gemäss Pfeilrichtung X aus der röhrchenförmigen Sonde 40 herausgeschobenen Zugglied 20', welches mit den beiden Einhängeteilen 23 an der Regenbogenhaut 13 eingehängt ist. Die röhrchenförmige Sonde 40 ist mit dem in Pfeilrichtung X verschobenen Halteelement 25 an der äusseren Kontur des Auges 10 beziehungsweise am Übergang 17 (Limbus) fixiert und gehalten. Bei zurückgezogener Regenbogenhaut 13 wird das Zugglied 20' entweder durch die Reibung der beiden Führungskörper 21 an der Innenwand der röhrchenförmigen Sonde 40 oder aber durch ein zweites gemäss Pfeilrichtung X an den Führungskörpern 21 verschiebbares Stopperelement 45 an der zugewandten Stirnseite (nicht bezeichnet) der Sonde 40 fixiert und gehalten. Hierdurch wird ein selbsttätiges Zurückziehen der eingehängten Führungskörper 21 in die Vorderkammer V verhindert.

Bei der Ausführungsform gemäss der Figuren 14 bis 15 kann zusätzlich zu dem jeweils kontrastfarbenen Zugglied 20 auch die Sonde 40 aus einem in Bezug auf die Regenbogenhaut 13 homogen und kontrastfarben eingefärbten Röhrchen hergestellt werden. Hierbei ist es wesentlich und zweckmässig, dass mindestens der in eingeführtem Zustand über der Regenbogenhaut 13 liegende Teil des Führungskörpers 21 und/oder das entsprechende Teilstück der röhrchenförmigen Sonde 40 in Bezug auf die Regenbogenhaut 13 kontrastfarben ausgebildet ist.

Das an dem Zugglied 20 beziehungsweise an der röhrchenförmigen Sonde 40 verschiebbar und klemmend angeordnete Halteelement 25 beziehungsweise das Stopperelement 45 (Fig.17,18) sind jeweils aus einer optisch signalfarben eingefärbten Kautschukmischung, beispielsweise aus einer eingefärbten Silikon-Kautschukmischung hergestellt.

Weitere zweckmässige Ausgestaltungen und Anordnungen der einzelnen Funktionselemente, die Wahl der Materialien und der in Bezug auf die Regenbogenhaut kontrastfarbenen Gestaltung sind ebenfalls möglich, ohne den Grundgedanken der Erfindung zu verlassen.

## Patentansprüche

1. Vorrichtung für den operativen Eingriff am Auge eines Lebewesens, mit einem in die Vorderkammer (V) des Auges (10) einführbaren und zum Zurückziehen der Regenbogenhaut (13) mit mindestens einem hakenförmig ausgebildeten Einhängeteil (23) versehenen Zugglied (20;20') und einem in Längsrichtung daran verschiebbar angeordneten und mit der äusseren Kontur des Auges anliegend in Eingriff bringbaren Halteelement, **dadurch gekennzeichnet, dass** mindestens das vordere als Einhängeteil (23) ausgebildete Teilstück des Zuggliedes (20;20') aus verformbarem Material mit Formgedächtniseffekt hergestellt ist, und dass das Zugglied (20;20') mit weitgehend geradlinig aufgebogenem Einhängeteil (23) in die Vorderkammer (V) einführbar ist und darin infolge der eigenen Materialeigenschaften zum Einhängen und Zurückziehen der Regenbogenhaut (13) wieder in die vorgegebene hakenförmige Form zurückführbar ist.

2. Vorrichtung nach Anspruch 1, **gekennzeichnet durch** eine in die Vorderkammer (V) einführbare längliche Sonde (40) und ein mit weitgehend geradlinig aufgebogenem Einhängeteil (23) derart in der Sonde (40) angeordnetes Zugglied (20), dass bei einer in axialer Richtung orientierten Relativbewegung des Zuggliedes (20) in Bezug auf die Sonde (40) oder umgekehrt das Einhängeteil (23) ausser Eingriff der Sonde (40) gelangt und infolge der Materialeigenschaften zum Einhängen und Zurückziehen der Regenbogenhaut (13) in die hakenförmige Form zurückführbar ist.

3. Vorrichtung nach Anspruch 1, **gekennzeichnet durch** eine in die Vorderkammer (V) einführbare längliche Sonde (40) sowie ein mit zwei weitgehend geradlinig aufgebogenen Einhängeteilen (23) versehenes und derart in der Sonde (40) angeordnetes Zugglied (20'), dass bei einer in axialer Richtung orientierten Relativbewegung des Zuggliedes (20') in Bezug auf die Sonde (40) oder umgekehrt jedes Einhängeteil (23) ausser Eingriff der Sonde (40) gelangt und infolge der Materialeigenschaften zum Einhängen und Zurückziehen der Regenbogenhaut (13) in die hakenförmige und etwa Λ-gespreizte Form zurückführbar ist.

4. Vorrichtung nach einem der Ansprüche 1 bis 3, **dadurch gekennzeichnet, dass** mindestens das Einhängeteil (23) des einzelnen Zuggliedes (20;20') zum weitgehend geradlinigen Aufbiegen sowie zum selbsttätigen Zurückführen in die vorgegebene hakenförmige Form aus flexiblem, polymerem Material mit thermischem oder mechanischem Formgedächtniseffekt hergestellt ist.

5. Vorrichtung nach einem der Ansprüche 1 bis 3, **dadurch gekennzeichnet, dass** das Zugglied (20;20') mit dem hakenförmig angeformten und weitgehend geradlinig aufbiegbaren Einhängeteil (23) über die gesamte Länge in Form eines flexiblen Filaments aus polymerem Material mit thermischem oder mechanischem Formgedächtniseffekt hergestellt ist.

6. Vorrichtung nach einem der Ansprüche 1 bis 3, **dadurch gekennzeichnet, dass** mindestens das Einhängeteil (23) des einzelnen Zuggliedes (20;20') zum weitgehend geradlinigen Aufbiegen sowie zum selbsttätigen Zurückführen in die vorgegebene hakenförmige Form aus flexiblem Metall, beispielsweise aus einer Nickel-Titan Legierung mit thermischem oder mechanischem Formgedächtniseffekt hergestellt ist.

7. Vorrichtung nach einem der Ansprüche 1 bis 3, **dadurch gekennzeichnet, dass** das Zugglied (20;20') mit dem hakenförmig angeformten und weitgehend geradlinig aufbiegbaren Einhängeteil (23) über die gesamte Länge in Form eines Filaments aus flexiblem Metall, beispielsweise aus einer Nickel-Titan Legierung mit thermischem oder mechanischem Formgedächtniseffekt hergestellt ist.

8. Vorrichtung nach Anspruch 1, **dadurch gekennzeichnet, dass** das Einhängeteil (23) des einzelnen Zuggliedes (20;20') bei einer in Bezug auf die Temperatur in der Vorderkammer (V) tieferen Temperatur geradlinig aufbiegbar und nach dem Einführen in die Vorderkammer (V) infolge der höheren Temperatur und des Formgedächtniseffektes in die hakenförmige Form zurückführbar ist.

9. Vorrichtung nach Anspruch 8, **dadurch gekennzeichnet, dass** das Einhängeteil (23) des einzelnen Zuggliedes (20;20') bei einer Raumtemperatur von etwa 18°C bis 22°C geradlinig aufbiegbar und nach dem Einführen in die Vorderkammer (V) infolge der höheren Körpertemperatur von etwa 35°C bis 37°C und des Formgedächtniseffektes in die hakenförmige Form zurückführbar ist.

10. Vorrichtung nach Anspruch 1, **dadurch gekennzeichnet, dass** das Zugglied (20;20') ausgehend von dem hakenförmigen Einhängeteil (23) ein in axialer Richtung sich mindestens über die zurückzuziehende Regenbogenhaut (13) erstreckendes und optisch signalfarben eingefärbtes Teilstück aufweist.

11. Vorrichtung nach Anspruch 1, **dadurch gekennzeichnet, dass** das einzelne Zugglied (20;20') über die gesamte Länge optisch signalfarben eingefärbt ist.

12. Vorrichtung nach Anspruch 1, **dadurch gekennzeichnet, dass** das Zugglied (20;20') über die gesamte Länge aus einem in Bezug auf die Farbe der jeweiligen Regenbogenhaut homogen und kontrastfarben eingefärbten flexiblen Filament aus polymerem oder thermoplastischem Material mit thermischem oder mit mechanischem Formgedächtniseffekt hergestellt ist.

13. Vorrichtung nach den Ansprüchen 2 und 3, **dadurch gekennzeichnet, dass** die in die Vorderkammer (V) einführbare Sonde (40) sowie das darin angeordnete Zugglied (20;20') jeweils ein in axialer Richtung sich mindestens über die zurückzuziehende Regenbogenhaut (13) erstreckendes und optisch signalfarben eingefärbtes Teilstück aufweist.

14. Vorrichtung nach den Ansprüchen 2 und 3, **dadurch gekennzeichnet, dass** die in die Vorderkammer (V) einführbare Sonde (40) sowie das darin angeordnete Zugglied (20;20') jeweils über die gesamte Länge aus optisch signalfarben eingefärbtem Material hergestellt ist.

15. Vorrichtung nach Anspruch 14, **dadurch gekennzeichnet, dass** die in die Vorderkammer (V) einführbare Sonde (40) sowie das darin angeordnete Zugglied (20;20') jeweils über die gesamte Länge aus einem in Bezug auf die Farbe der jeweiligen Regenbogenhaut homogen und kontrastfarben eingefärbten Material in Form eines Filaments oder Fadens hergestellt ist.

16. Vorrichtung nach Anspruch 1, **dadurch gekennzeichnet, dass** das an dem Zugglied (20;20') in Längsrichtung verschiebbar und selbstklemmend angeordnete und scheibenförmig ausgebildete Halteelement (25) aus einer optisch signalfarben eingefärbten Silikon-Kautschukmasse hergestellt ist.

17. Vorrichtung nach den Ansprüchen 2 und 3, **dadurch gekennzeichnet, dass** an der Sonde (40) ein erstes in Längsrichtung verschiebbares Halteelement (25) und an dem geraden aus der Sonde (40) ragenden Ende des darin geführten Zuggliedes (20;20') ein relativ zu der Sonde (40) verschiebbares Stopperelement (45) angeordnet ist, und dass die beiden Elemente (25;45) jeweils aus einer optisch signalfarben eingefärbten Silikon-Kautschukmasse hergestellt sind.

18. Vorrichtung nach Anspruch 1, **dadurch gekennzeichnet, dass** in die Hornhaut (15) zum Einführen des Zuggliedes (20;20') ein Hülsenkörper (30) eingesetzt ist, welcher ein bis in die Vorderkammer (V) ragendes und beim Herausziehen des Zuggliedes (20;20') als Gegenlager für das aufzubiegende Einhängeteil (23) vorgesehenes Röhrchen (31) aufweist.

19. Vorrichtung nach Anspruch 1, **gekennzeichnet durch**
**a)** die Verwendung eines aus verformbarem Material, beispielsweise aus polymerem Material oder Metall in Form eines flexiblen Filaments mit thermischem oder mechanischem Formgedächtniseffekt hergestellten sowie mit einem hakenförmigen Einhängeteil (23) versehenen Zuggliedes (20;20'), bei welchem
**b)** das Einhängeteil (23) in einer ersten Phase etwa geradlinig aufgebogen in die Vorderkammer (V) des Auges (10) eingeführt und in einer zweiten Phase infolge der Materialeigenschaften selbsttätig in die hakenförmige Formgebung zum Einhängen und Zurückziehen der Regenbogenhaut (13) zurückgeführt wird, wobei
**c)** das einzelne an der Regenbogenhaut (13) eingehängte Zugglied (20;20') mittels des daran verschiebbar angeordneten Halteelements (25) an der äusseren Kontur des Auges (10) fixierbar ist.

20. Vorrichtung nach den Ansprüchen 2 und 3, **gekennzeichnet durch folgende Merkmale:**
**a)** Anordnung des mit mindestens einem hakenförmigen Einhängeteil (23) versehenen Zuggliedes (20;20') aus verformbarem Material, beispielsweise aus polymerem Material oder Metall in Form eines flexiblen Filaments mit thermischem oder mechanischem Formgedächtniseffekt in eine längliche, röhrchenförmige Sonde (40),
**b)** Einführen der länglichen Sonde (40) zusammen mit dem koaxial darin angeordneten Zugglied (20;20') in die Vorderkammer (V) des Auges (10),
**c)** Relativbewegung des Zuggliedes (20;20') in Bezug auf die Sonde (40) oder umgekehrt für die Freigabe und gleichzeitige Rückbildung des aufgebogenen Einhängeteils (23) in die ursprüngliche hakenförmige Form,
**d)** Einhängen und Zurückziehen der Regenbogenhaut (13) mittels des aus der Sonde (40) ragenden und mit dem Einhängeteil (23) versehenen Zuggliedes (20;20'),
**e)** Fixieren der Sonde (40) mittels des verschiebbaren Halteelements (25) an der äusseren Kontur des Auges (10) sowie des Zuggliedes (20;20') mittels des verschiebbaren Stopperelements (45) an der zugewandten Stirnseite der Sonde (40), und
**f)** Entfernen des Einhängeteils (23) in Bezug auf die Regenbogenhaut (13) und Zurückziehen des Zuggliedes (20;20') in die Sonde (40) zum Herausziehen aus der Vorderkammer (V) des Auges (10).
